# EUROPÄISCHE PATENTSCHRIFT

(11) **EP 0 798 297 B1**
(45) Veröffentlichungstag und Bekanntmachung des Hinweises auf die Patenterteilung: **31.10.2001**
(21) Anmeldenummer: 97105061.2
(22) Anmeldetag: 25.03.1997
(51) Int. Cl.: C07D 239/52

(54) **Verfahren zur Herstellung von 2-Alkoxy-6-(trifluormethyl)-pyrimidin-4-ol**
Process for the preparation of 2-alkoxy-6-(trifluoromethyl)-pyrimidine-4-ol
Procédé pour la préparation de 2-alkoxy-6-(trifluormethyl)-pyrimidine-4-ol

(30) Priorität: 26.03.1996 CH 78096; 11.07.1996 CH 174096
(43) Veröffentlichungstag der Anmeldung: 01.10.1997
(73) Patentinhaber: Lonza AG, 3930 Visp (CH)
(72) Erfinder: Schmidt, Beat, Dr., 3937 Baltschieder (Kanton Wallis) (CH); Stucky, Gerhard, Dr., 3902 Brig-Glis (Kanton Wallis) (CH)
(74) Vertreter: Winter, Brandl, Fürniss, Hübner, Röss, Kaiser, Polte Partnerschaft

(56) Entgegenhaltungen:
- EP-A- 0 603 893
- WO-A-96/16047
- CH-A- 685 497
- GB-A- 1 296 371
- US-A- 3 966 730
- TETRAHEDRON (TETRAB,00404020);84; VOL.40 (17); PP.3313-20, UNIV. "LA SAPIENZA";DEP. CHEM.; ROME; 00185; ITALY (IT), XP002034345 BOTTA M ET AL: "6-Alkyl and 5,6-dialkyl-2-methoxy-4(3H)-pyrimidinones in the transformations of pyrimidines - 2. Synthesis and conversion into alkyluracils and 2-alkoxy-4(3H)-pyrimidinones"
- J. MED. CHEM., Bd. 39, Nr. 8, 12.April 1996, Seiten 1720-1728, XP002034346 FELCZAK ET AL.: "6-Substituted and 5,6-disubstituted derivatives of uridine ..."
- J. HETEROCYCL. CHEM., Bd. 9, Nr. 3, 1972, Seiten 513-522, XP000655246 LUTZ ET AL.: "Novel 6-trifluoromethylcytosines and ..."

## Beschreibung

Die Erfindung betrifft ein neues Verfahren zur Herstellung von 2-Alkoxy-6-(trifluormethyl)-pyrimidin-4-ol ausgehend von Cyanamid.

2-Alkoxy-6-(trifluormethyl)-pyrimidin-4-ol wie 2-Isopropoxy-6-(trifluormethyl)-pyrimidin-4-ol sind wichtige Zwischenprodukte zur Herstellung von Insektiziden (EP-A 0 407 873).

A.W. Lutz und S.H. Trotto [J. Het. Chem., 9:513-522, (1972)] beschreiben die Herstellung von 2-Isopropoxy-6-(trifluormethyl)-pyrimidin-4-ol durch Alkylierung von 6-(Trifluormethyl)-uracil mit Isopropylbromid. M. Botta et al. [Tetrahedron 40:3313-3320, (1984)] beschreiben die Herstellung von 6-Alkyl-2-methoxypyrimidonen ausgehend von O-Methylisoharnstoffbisulfat und dem entsprechenden β-Ketoester in Gegenwart von Calciumhydroxid in einer Wasser/Ethanol-Lösung, und die Herstellung von 2-Methoxy-6-(trifluormethyl)-pyrimidin-4-ol aus Methylisoharnstoff und Trifluoracetessigsäureethylester in Gegenwart von Calciumhydroxid in einer Mischung aus Wasser und Ethanol wird von K.Felczak et al. [J. Med.Chem., 39:1720-1728 (1996)] beschrieben. Ähnliche Verfahren, bei denen nur Alkohol als Lösungsmittel verwendet wird, werden auch in der GB-A-1 296 371, der US-A-3 966 730 und derWO-A-96/16047 offenbart.

Aus der CH-PS 685 497 und der EP-A 0 603 893 ist die Herstellung von 2-Ethoxy-4,6-dihydroxypyrimidin bekannt. Dabei wird Cyanamid mit Ethanol und Chlorwasserstoff in das entsprechende Isoharnstoffderivat überführt, welches dann mit Malonsäurediethylester in Gegenwart von Natriumethylat, in Ethanol, zum Endprodukt umgesetzt wird.

Nachteile bei diesen Ietztgenannten Verfahren sind zum einen relativ lange Umsetzungszeiten und zum anderen, dass das dabei gebildete NaCl abfiltriert werden muss.

Aufgabe der vorliegenden Erfindung war es, ein wirtschaftlicheres Verfahren zur Herstellung von 2-Alkoxy-6-(trifluormethyl)-pyrimidin-4-ol zur Verfügung zu stellen, welches in wesentlich kürzerer Zeit und ohne NaCl-Filtration durchgeführt werden kann.

Diese Aufgabe wird mit dem Verfahren gemäss Patentanspruch I und Patentanspruch 7 gelöst.

Erfindungsgemäss wird das Verfahren derart durchgeführt, dass man in der ersten Stufe Cyanamid mit einem C₁-C₈-Alkohol in Gegenwart von Chlorwasserstoff zum entsprechenden Alkoxyisohamstoffhydrochlorid umsetzt und dieses dann in der zweiten Stufe mit Trifluoracetessigsäureethylester, in Wasser, in Gegenwart eines Alkalimetallhydroxids zum Endprodukt gemäss der allgemeinen Formel 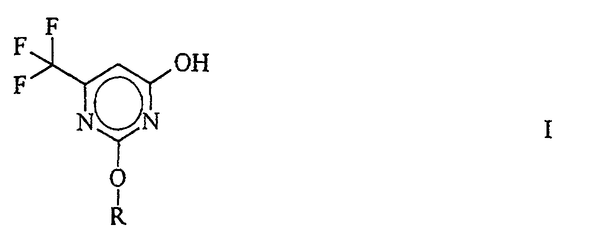 worin R eine C₁-C₈-Alkylgruppe bedeutet, umsetzt.

Als Alkylimetallhydroxid kann Natrium- oder Kaliumhydroxid, vorzugsweise Natriumhydroxid, verwendet werden. Vorzugsweise wird das Alkalimetallhydroxid aequimolar zum Trifluoracetessigsäureethylester eingesetzt.

Als C₁-C₈-Alkohol können Methanol, Ethanol, Propanol, i-Propanol, Butanol, i-Butanol, Pentanole, Hexanole, Heptanole oder Octanole eingesetzt werden. Vorzugsweise wird i-Propanol, Propanol oder i-Butanol eingesetzt.

Zweckmässig wird die Umsetzung in der ersten Stufe bei einer Temperatur von 0 bis 100 °C, vorzugsweise bei einer Temperatur von 50 bis 70 °C, durchgeführt.
Die zweite Stufe wird zweckmässig bei einer Temperatur von 50 bis 100 °C, vorzugsweise von 85 bis 95 °C, durchgeführt.

Die Umsetzung kann sowohl mit Isolation des entsprechenden Alkoxyisoharnstoffhydrochlorids als auch ohne dessen Isolation durchgeführt werden. Vorzugsweise wird die Umsetzung ohne Isolation des Alkoxyisoharnstoffhydrochlorids durchgeführt.

Dabei beträgt die Gesamtumsetzungszeit zwischen 6 und 8 h, wobei das während der Umsetzung gebildete NaCl in Wasser gelöst ist und demnach nicht filtriert werden muss.

### Beispiel 1

### Herstellung von 2-Isopropoxy-6-(trifluormethyl)-pyrimidin-4-ol

11,25 g (263 mmol; 1,05 Aeq.) Cyanamid wurden in Isopropanol (800 ml pro mol Trifluoracetessigsäureethylester; TFAAEt) bei 50 °C vorgelegt und HCI-Gas (18,3 g; 500 mmol; 2,0 Aeq.) während einer Stunde durchgeleitet. Nach Beendigung des HCl-Einleitens wurde 2 h lang bei 70 °C gerührt. Danach liess man auf Raumtemperatur abkühlen und dampfte ¾ des Lösungsmittels am Rotationsverdampfer ein. 80 ml Wasser (320 ml pro mol Trifluoracetessigsäureethylester) wurden zum gebildeten O-Isopropylisohamstoff-hydrochlorid bei Raumtemperatur zugegeben. Anschliessend fügte man bei Raumtemperatur innerhalb von 5 min eine wässrige NaOH-Lösung (78,87 g; 1,09 Aeq.; 14%ig nach Titration) zu, bis der pH von 0,5 auf 11,5 verschoben war und liess weitere 15 min rühren. Während der NaOH-Zugabe wurde die Temperatur ≤30 °C gehalten. Zu dieser Lösung wurde danach bei Raumtemperatur Trifluoracetessigsäureethylester (46,5 g; 500 mmol; 1,0 Aeq.) zugetropft. Dabei stieg die Temperatur auf 30 °C an und es bildeten sich zwei Phasen. Dieses Gemisch wurde für 2 h auf Rückfluss erhitzt. Die organischen Lösungsmittel wurden dann bis zu einer Kopftemperatur von ca. 96 °C abdestilliert. Man liess weitere 2 h bei 85 - 90 °C rühren. Die leicht gelbe Emulsion liess man auf Raumtemperatur abkühlen, stellte mit wenig wässriger 2N HCl-Lösung den pH von 7,3 auf 5,0 ein und filtrierte den granulatförmigen Feststoff nach 20 min bei Raumtemperatur ab. Der nach der Filtration erhaltene feuchte Feststoff wurde erneut in destilliertem Wasser aufgenommen und 1 h auf Rückfluss erhitzt. Nach Abkühlen auf Raumtemperatur, Filtration und Trocknung erhielt man einen leicht gelblichen Feststoff mit höherem Gehalt: Gesamtausbeute 67,8% (98,0%ig nach HPLC). Man wusch den Feststoff mit wenig kaltem Wasser nach und trocknete anschliessend über Nacht im Vakuumtrockenschrank bei 40 °C.
Es wurden 41,3 g gelblicher Feststoff als Produkt entsprechend einer Ausbeute von 69,8% (Gehalt: 94,5%ig nach HPLC) erhalten.

### Beispiel 2

### Herstellung von 2-Propoxy-6-(trifluormethyl)-pyrimidin-4-ol

1,05 Aeq. Cyanamid wurde in Propanol (800 ml pro Mol TFAAEt) bei 50 °C vorgelegt und 2,0 Aeq. HCI-Gas während 0,5 Stunden durchgeleitet (exotherm, leichte Wasser-Kühlung). Anschliessend wurde die Lösung auf 70 °C erwärmt und 2 Stunden bei dieser Temperatur gehalten. Danach liess man auf Raumtemperatur abkühlen und dampfte 2/3 des Lösungsmittels am Rotationsverdampfer ein. 32 ml Wasser (320 ml pro Mol TFAAEt) wurde zum O-Propylisoharnstoff-hydrochlorid bei Raumtemperatur zugegeben. Anschliessend fügte man bei Raumtemperatur innerhalb von 7 min eine wässrige NaOH-Lösung (14%ig nach Titration) zu, bis der pH von 0 auf 11,5 verschoben war (Temp. ≤30 °C; entspricht 1,1 Aeq. NaOH) und liess weitere 15 min rühren. Zu dieser Lösung wurde danach innerhalb von 2 min 1,0 Aeq. TFAAEt zugetropft. Dabei stieg die Temperatur auf 32 °C an und es bildeten sich zwei Phasen. Dieses Gemisch wurde für 2 Stunden auf Rückfluss
(~88 °C) erhitzt. Die organischen Lösungsmittel wurden dann bis zu einer Kopftemperatur ≅ 95 °C abdestilliert. Man liess weitere 2 Stunden bei 84 - 85 °C rühren. Die leicht gelbe Emulsion liess man auf Raumtemperatur abkühlen, stellte mit wenig konz. HCl-Lösung den pH auf 5,0 ein. Man kühlte die Suspension über Nacht auf 4 °C. Der granulatförmige Feststoff wurde abfiltriert, wusch den Feststoff mit wenig kaltem Wasser nach und trocknete anschliessend über Nacht im Vakuumtrockenschrank bei 40 °C.
- Ausbeute:: 13,1 g, klebriger Feststoff. 52,36% (Gehalt: 88,8%ig nach GC FI%).
- ¹H-NMR:: 1,0 (*l*, 3H, CH₃),
1,8 (*m*, 2H, CH₂),
4,4 (*m*, 2H, CH₂),
6,5 (*s*, 1H, ar-H),
12 (*s*, 1H, OH).

### Beispiel 3

### Herstellung von 2-Isobutoxy-6-(trifluormethyl)-pyrimidin-4-ol

1,05 Aeq. Cyanamid wurde in Isobutanol (800 ml pro Mol TFAAEt) bei 50 °C vorgelegt und 2,0 Aeq. HCl-Gas während 0,5 Stunden durchgeleitet (exotherm, leichte Wasser-Kühlung). Anschliessend wurde die Lösung auf 70 °C erwärmt und 2 Stunden bei dieser Temperatur gehalten. Danach liess man auf Raumtemperatur abkühlen und dampfte das Lösungsmittel am Rotationsverdampfer ein. 32 ml Wasser (320 ml pro Mol TFAAEt) wurde zum O-Isobutylisoharnstoff-hydrochlorid bei Raumtemperatur zugegeben. Anschliessend fügte man bei Raumtemperatur innerhalb von 9 min eine wässrige NaOH-Lösung (14%ig nach Titration) zu, bis der pH von 0 auf 11,5 verschoben war (Temp. ≤30 °C; entspricht 1,8 Aeq. NaOH) und liess weitere 15 min rühren. Zu dieser Lösung wurde danach innerhalb von 2 min 1,0 Aeq. TFAAEt zugetropft. Dabei stieg die Temperatur auf 30 °C an und es bildeten sich zwei Phasen Dieses Gemisch wurde für 2 Stunden auf Rückfluss (-90 °C -92 °C) erhitzt. Die organischen Lösungsmittel wurden dann bis zu einer Kopftemperatur ≅ 93 °C abdestilliert. Man liess weitere 2 Stunden bei 85 - 86 °C rühren. Die leicht gelbe Emulsion liess man auf Raumtemperatur abkühlen, stellte mit wenig konz. HCI-Lösung den pH von 8,5 auf 5,0. Man liens die Suspension über Nacht bei 4 °C stehen. Man filtrierte den granulatförmigen Feststoff ab, wusch den Feststoff mit wenig kaltem Wasser nach und trocknete anschliessend über Nacht im Vakuumtrockenschrank bei 40 °C.
- Ausbeute:: 14.09 g gelbes Öl. 59,6% (Gehalt: 85,3%ig nach GC Fl%).
- ¹H-NMR:: 1,0 (*d*, 6H, CH₃),
2,1 (*m*, 2H, CH),
4,2 (*d*, 2H, CH₂),
6,5 (*s*, 1H, ar-H),
12 (*s*, 1H, OH).

## Patentansprüche

1. Verfahren zur Herstellung von 2-Alkoxy-6-(trifluormethyl)-pyrimidin-4-ol der allgemeinen Formel 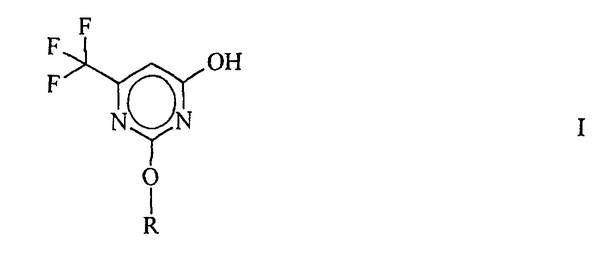 worin R eine C₁-C₈-Alkylgruppe bedeutet, **dadurch gekennzeichnet, dass** man in der ersten Stufe Cyanamid mit einem C₁-C₈-Alkohol in Gegenwart von Chlorwasserstoff zum entsprechenden Alkoxyisoharnstoffhydrochlorid umsetzt und dieses dann in der zweiten Stufe mit Trifluoracetessigsäureethylester, in Wasser, in Gegenwart eines Alkalimetallhydroxids zum Endprodukt gemäss Formel I überführt.

2. Verfahren nach Patentanspruch 1, **dadurch gekennzeichnet, dass** man als Alkalimetallhydroxid Natriumhydroxid einsetzt.

3. Verfahren nach mindestens einem der Patentansprüche 1 bis 2, **dadurch gekennzeichnet, dass** man das Alkalimetallhydroxid aequimolar zum Trifluoracetessigsäureethylester einsetzt.

4. Verfahren nach mindestens einem der Patentansprüche 1 bis 3, **dadurch gekennzeichnet, dass** man die Umsetzung in der ersten Stufe bei einer Temperatur von 0 bis 100 °C durchführt.

5. Verfahren nach mindestens einem der Patentansprüche 1 bis 4, **dadurch gekennzeichnet, dass** man die Umsetzung in der zweiten Stufe bei einer Temperatur von 50 bis 100 °C durchführt.

6. Verfahren nach mindestens einem der Patentansprüche 1 bis 5, **dadurch gekennzeichnet, dass** man die Umsetzung ohne Isolation des Alkoxyisoharnstoffhydrochlorids durchführt.

7. Verfahren zur Herstellung von 2-Alkoxy-6-(trifluormethyl)-pyrimidin-4-ol der allgemeinen Formel 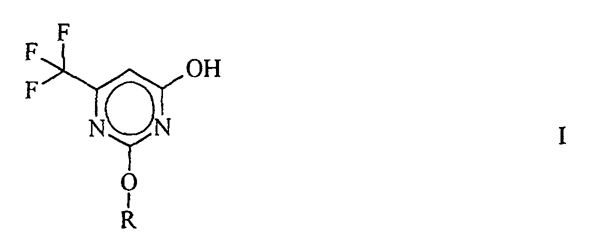 worin R die in Anspruch 1 genannte Bedeutung hat, **dadurch gekennzeichnet, dass** man das entsprechende Alkoxyisoharnstoffhydrochlorid mit Trifluoracetessigsäureethylester, in Wasser, in Gegenwart eines Alkalimetallhydroxids, umsetzt.

## Claims

1. Process for preparing 2-alkoxy-6-(trifluoromethyl)-pyrimidin-4-ol corresponding to the general formula 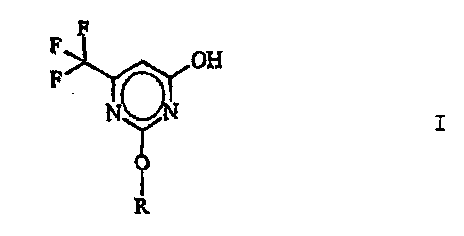 wherein R denotes a C₁-C₈-alkyl group, **characterised in that** in the first step, cyanamide is reacted with a C₁-C₈-alcohol in the presence of hydrogen chloride to form the corresponding alkoxyisourea hydrochloride and this, in the second step, is then converted by means of ethyl trifluoroacetoacetate, in water, in the presence of an alkali metal hydroxide, into the end product corresponding to formula I.

2. Process according to claim 1, **characterised in that** the alkali metal hydroxide used is sodium hydroxide.

3. Process according to at least one of claims 1 and 2, **characterised in that** the alkali metal hydroxide is used in an equimolar proportion to the ethyl trifluoroacetoacetate.

4. Process according to at least one of claims 1 to 3, **characterised in that** the reaction in the first step is carried out at a temperature of 0 to 100°C.

5. Process according to at least one of claims 1 to 4, **characterised in that** the reaction in the second step is carried out at a temperature of 50 to 100°C.

6. Process according to at least one of claims 1 to 5, **characterised in that** the reaction is carried out without isolation of the alkoxyisourea hydrochloride.

7. Process for preparing 2-alkoxy-6-(trifluoromethyl)-pyrimidin-4-ol corresponding to the general formula 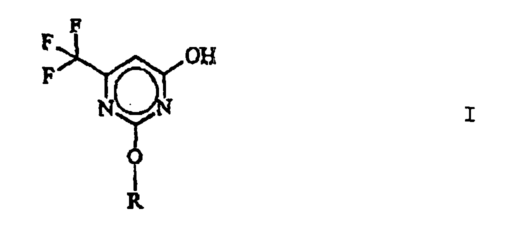 wherein R has the meaning given in claim 1, **characterised in that** the corresponding alkoxyisourea hydrochloride is reacted with ethyl trifluoroacetoacetate, in water, in the presence of an alkali metal hydroxide.

## Revendications

1. Procédé pour la préparation de 2-alcoxy-6-(trifluorométhyl)-pyrimidin-4-ol de la formule générale 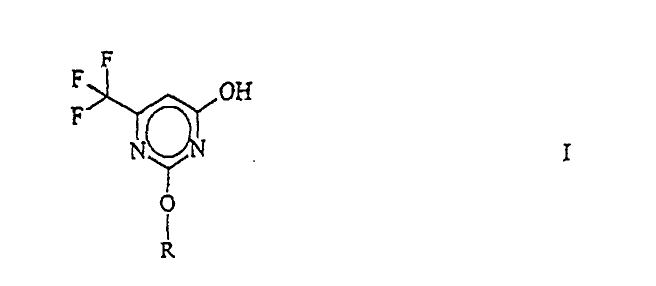 dans laquelle R signifie un groupe alkyle en C₁-C₈, **caractérisé en ce que** dans la première étape on transforme du cyanamide avec un alcool en C₁-C₈ en présence de chlorure d'hydrogène en le chlorhydrate d'alcoxyiso-urée correspondant et ensuite dans la deuxième étape avec l'éthylester de l'acide trifluoro-acétique, dans l'eau, en présence d'un hydroxyde des métaux alcalins on transforme en le produit final selon la formule 1.

2. Procédé selon la revendication 1, **caractérisé en ce qu'**en tant qu'hydroxyde des métaux alcalins on utilise de l'hydroxyde de sodium.

3. Procédé selon au moins l'une des revendications 1 à 2, **caractérisé en ce que** l'on utilise l'hydroxyde des métaux alcalins de façon équimolaire à l'éthylester de l'acide trifluoro-acétique.

4. Procédé selon au moins l'une des revendications 1 à 3, **caractérisé en ce que** l'on conduit la réaction dans la première étape à une température de 0 à 100°C.

5. Procédé selon au moins l'une des revendications 1 à 4, **caractérisé en ce que** l'on conduit la réaction dans la seconde étape à une température de 50 à 100 °C.

6. Procédé selon au moins l'une des revendications 1 à 5, **caractérisé en ce que** l'on conduit la réaction sans isolation du chlorhydrate d'alcoxyiso-urée.

7. Procédé pour la préparation de 2-alcoxy-6-(trifluorométhyl)-pyrimidin-4-ol de la formule générale 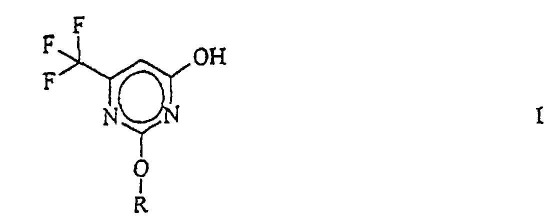 dans laquelle R a la signification citée dans la revendication 1, **caractérisé en ce que** l'on utilise le chlorhydrate d'alcoxyiso-urée correspondant avec l'éthylester de l'acide trifluoro-acétique, dans l'eau, en présence d'un hydroxyde des métaux alcalins.
